Europäisches Patentamt

European Patent Office

Office européen des brevets

(11) Publication number : **0 195 541**
**B1**

# EUROPEAN PATENT SPECIFICATION

(45) Date of publication of patent specification :
06.07.88

(51) Int. Cl.⁴ : **C 07 C  5/50, C 07 C  15/24, B 01 J  27/04, B 01 J  31/02**

(21) Application number : 86301404.9

(22) Date of filing : 27.02.86

(54) Process for conversion of alicyclic compounds into aromatic compounds.

(30) Priority : 18.03.85 US 713214

(43) Date of publication of application :
24.09.86 Bulletin 86/39

(45) Publication of the grant of the patent :
06.07.88 Bulletin 88/27

(84) Designated contracting states :
AT BE CH DE FR GB IT LI NL

(56) References cited :
US-A- 2 953 513
US-A- 3 666 687
C.J. COLLINS et al.: "Organic chemistry of coal", chapter 12, American Chemical Society, Washington, D.C., US; "Isotopic studies of thermally induced reactions of coal and coal-like structures"

(73) Proprietor : **MOBIL OIL CORPORATION**
150 East 42nd Street
New York New York 10017 (US)

(72) Inventor : **Rudnick, Leslie Robert**
5 Winthrop Road
Lawrenceville New Jersey 08648 (US)

(74) Representative : **Cooper, John Anthony et al**
Mobil Court 3 Clements Inn
London WC2A 2EB (GB)

Jouve, 18, rue St-Denis, 75001 Paris, France

## Description

This invention relates to the conversion of alicyclic compounds into aromatic compounds in a process in which the alicyclic compounds function as hydrogen donors. The invention relates more particularly to a process for converting alicyclic compounds into polynuclear, fused ring aromatic compounds and to hydrogen transfer process using alicyclic hydrogen donors.

Many processes are known for the dehydrogenation of alicyclic compounds to convert them to the corresponding aromatic compounds. For example, cyclic hydrocarbons may be dehydrogenated over various metal catalysts to form the corresponding aromatics but many of these processes are vapor phase processes which may be unsuitable for use with high boiling materials or, alternatively, the catalyst may be one which is sensitive to poisoning by compounds containing oxygen, nitrogen or sulfur atoms. There is, therefore, a continuing need for novel aromatization processes to be developed.

The use of various polycyclic compounds such as tetralin (tetrahydronaphthalene) as hydrogen donors in various processes is known. For example, certain coal liquefaction processes such as the Exxon Donor Solvent Process (described in Chem. Eng. Progr. 72, 145 (1976)) and some petroleum residuum treating processes employ donor solvents. Examples of residuum upgrading processes employing donor solvents are those described in U.S. Patents 4,292,168, 4,395,324, 4,090,947 and 3,238,118. Because the operation of these processes relies upon efficient transfer of hydrogen from the donor to the acceptor, it would be desirable to find ways of making the transfer more efficient, for example, by the use of catalysts. There is therefore a need for materials to catalyze the transfer of hydrogen from donors in donor solvent processes.

It has now been found that active sulfur containing compounds, especially thiols or thiophenols, are effective catalysts for the transfer of hydrogen from alicyclic compounds such as decalin, to hydrogen acceptors. At the same time, the alicyclic compound is converted into an aromatic compound in good yield. The reaction therefore provides a preparative route for aromatic compounds which may not be susceptible of convenient synthesis by other means.

According to the present invention, there is provided a process for the conversion of an alicyclic compound into an aromatic compound which comprises dehydrogenating the alicyclic compound in the presence of a hydrogen acceptor and a catalyst, characterized in that the catalyst is an organic, active sulfur-containing compound.

Generally, the reaction is carried out in the liquid phase using, if necessary, superatmospheric pressure to maintain the reactants in the liquid phase.

In the present process, alicyclic compounds are converted into the corresponding aromatic compounds by the use of an active sulfure compound as the catalyst. Alicyclic compounds are cyclic compounds which do not contain any aromatic rings fused to a non-aromatic ring. Thus, alicyclic compounds may be either compounds which do not contain any aromatic ring systems or, if an aromatic ring is present in the compound, it is not fused to a non-aromatic ring. Thus, aromatic ring structures may be present as substituents either on the ring itself or at a more distant part of the molecule, for example as in phenyl cyclohexane or 1-cyclohexyl-4-phenyl-butane. In the past, it was thought that the presence of a fused aromatic-naphtenic ring system as in tetralin was necessary to confer good donor properties. It has now been found, however, that certain catalysts containing active sulfur will effectively catalyze the transfer of hydrogen from materials which were not previously considered to be hydrogen donors. According to the present invention, alicyclic compounds function in this way.

The alicyclic compound will usually be one which contains a six-membered saturated or unsaturated homocyclic carbon ring system which, upon dehydrogenation, will form an aromatic ring system. Compounds of this kind may be simple alicyclic compounds such as cyclohexane or cyclohexene which may be converted into benzene by the present aromatization process but may also have a multiple ring structure as in decahydronaphthalene which may be converted into naphthalene or decahydropyrene which may be converted either into pyrene itself or its partially hydrogenated derivatives, for example 4,5-dihydropyrene or 4,5,9,10-tetrahydropyrene. Similarly, the partly hydrogenated derivatives of pyrene may be converted to pyrene itself. Substituted alicyclic compounds may also be converted to the corresponding aromatic compounds, especially where the substituent is a hydrocarbon group, for example an alkyl group such as methyl, ethyl, propyl, butyl or is itself an aromatic group, for example phenyl or a substituted alkyl group such as benzyl. In this way, methylcyclohexane may be converted into toluene ; methyl decahydronaphthalene may be converted into methylnaphthalene and other, more highly substituted derivatives of various alicyclic compounds may be converted into the corresponding aromatic forms by removal of the hydrogens from the alicyclic ring. Non-hydrocarbon substituents such as halo, for example chloro or bromo or sulfonate may also be present although, in most cases, the simple hydrocarbons substituents will be preferred.

The alicyclic compound may also be a heterocyclic compound, either with a simple ring system such as piperidine which is converted into pyrazine, or tetrahydroquinoline which is converted into quinoline. Again, hydrocarbon or non-hydrocarbon substituents may be present.

The reaction is carried out in the presence of the substrate which acts as a hydrogen acceptor. Generally, this substrate will be a liquid which is a mild oxidizing agent and which, under the reaction conditions, will combine with hydrogen from the alicyclic compound. When the process is being used for the purpose of obtaining an aromatic product from an alicyclic precursor, the hydrogen acceptor will be chosen according to various factors such as its efficacy as a hydrogen acceptor, the nature of the reduced product, for example so as to permit ready separation from the desired aromatic compound, and its cost. Bis-aromatic ketones such as benzophenone and substituted bis-aromatic ketones are often suitable acceptors, being readily converted into the corresponding hydrocarbons. On the other hand, when the process is being employed to hydrogenate a process stream by hydrogen transfer from the alicyclic compound as an H-donor, it will be the alicyclic compound that will be selected according to factors such as those described above. Process streams which may be hydrogenated by hydrogen transfer in this manner include petroleum residua and high boiling petroleum fractions such as heavy cycle oils, slurry oils, clarified slurry oils, FCC tower bottoms, coker feeds, and other petroleum refinery fractions typically boiling above 350 °C or even higher.

An active sulfur compound is also present during the reaction, that is, a compound which contains an —SH group. This may be an aliphatic thiol or mercaptan or an aromatic thiophenolic compound. Suitable aliphatic thiols include the alkyl thiols such as methylmercaptan, ethyl mercaptan, propyl mercaptan, or butyl mercaptan or substituted alkyl mercaptans. However, the simple alkyl mercaptans will be preferred since they are more stable at the elevated temperatures normally used in this reaction. The active sulfur compound may also be a thiophenolic compound, for example, thiophenol, 1-thionaphthol or 2-thionaphthol, a substituted thiophenol in which the substituent is, for example, halo such as chloro or bromo, or sulfonate, or a substituted thionaphthol.

The sulfur compound may be added to the alicyclic reactant and the hydrogen acceptor as such or in the form of a precursor which will be converted into a suitable catalyst under the reaction conditions employed. For example, it has been found that benzothiophene, although itself not an active sulfur containing compound, is effective to transfer hydrogen from alicyclic compounds to various hydrogen acceptors. It is believed that under appropriate conditions, hydrogen transfer to the benzothiophene occurs to produce a saturated sulfur-containing ring attached to the benzenoid nucleus and that this ring then opens to form a species containing an available active sulfur, probably as a free radical. By contrast, dibenzothiophene is inactive as an H-transfer catalyst; it is believed that this may be because ring opening in dibenzothiophene would be less favored as it would require the cleavage of a bond leading to the production of a phenyl radical.

The reaction is carried out at elevated temperatures in the liquid phase and temperatures of at least 200 °C or even higher, for example 400 °C or above may be necessary. The system may be maintained under autogenous pressure or superatmospheric pressure in order to maintain the reactants in the liquid phase.

The active sulfur compound or its precursor may conveniently be added to the other reactants as a constituent of a process stream, for example a petroleum refinery stream, which contains it. If this is done, there is no need to separate out the sulfur compound from the process stream. Because many petroleum refinery streams contain mercaptans, various thiophenolic compounds or their precursors, for example benzothiophene, petroleum refining operations provide a ready source of catalysts for the H-transfer process. The process stream selected may also contain other reactants, for example alicyclic compounds as hydrogen donors or hydrogen acceptors. Because the process may, in this way, effectively use different refinery process streams to provide reactants it is very effectively utilized in petroleum upgrading processes employing H-transfer reactions, for example, residuum upgrading processes such as donor visbreaking and donor cracking, as described in U.S. Patents 4,347,120 4,292,168, 4,178,229, 4,395,324, 4,292,168, 4,090,947, and 2,953,513 to which reference should be made for details of such processes. Similarly, it may be used in H-donor coal liquefaction processes such as the Donor Solvent Process and the liquefaction processes described in British Patents 1,597,119, 1,603,619 and 2,015,565.

The reaction may also be used for the production of specific aromatics from alicyclic precursors, for the production of aromatics in oils with alicyclic constituents, particularly where these alicyclic constituents contain carbocyclic rings formed by the cyclization of side chains on aromatic compounds and for the aromatization of naphthenic crudes or feedstocks, particularly feedstocks or fractions which are low in oxygen, nitrogen and sulfur for carbon fiber pitch production. Thus, if a petroleum refinery stream contains a significant proportion of alicyclic compounds which are to be converted into aromatics, the refinery stream may be treated with the active sulfur compound and a hydrogen acceptor added if there is no suitable compound already present in the stream, to convert the alicyclics into the desired aromatics. The method is particularly useful for the production of carbon fiber pitch which requires the presence of highly aromatic, fused ring compounds because here, treatment of a precursor refinery stream will ensure that the desired fused ring compounds are present to the maximum extent possible. A further utility is in the liquefaction of coal by the process described in copending application Serial No. 714,777 filed 22 March 1985.

The following Examples illustrate the invention.

## Example 1

Perhydropyrene was heated to 440 °C for one hour in the presence of benzophenone (0.42 molar ratio) as a model hydrogen acceptor. No pyrene was formed under these conditions.

In a separate experiment, the above reaction was carried out in the presence of a small amount (0.51 wt. %) of thiophenol. This resulted in a conversion of 94.7 % of benzophenone into diphenylmethane, indicating hydrogen transfer and the formation of pyrene as a product, confirmed by gas chromatography.

Examples 2-5

Perhydropyrene (PHP) as a hydrogen donor was heated with benzophenone (0.42 molar ratio) as a model hydrogen acceptor in the presence of various active sulfur containing catalysts. The reaction was carried out at 454 °C for 2.5 minutes equal to a severity of 800 seconds ERT (Equivalent Reaction Time). The results are given in Table 1 below, with the hydrogen donor efficiency, HDonEff, reported as the number of millimoles of hydrogen transferred per gram of sample H-donor.

Table 1

PHP with Sulfur H-transfer Catalysts

| Example | Catalyst | HDonEff |
|---|---|---|
| 2 | $n\text{-}C_{12}H_{25}SH$ | 1.22 |
| 3 | Thiophenol | 3.37 |
| 4 | 1-thionaphthol | 0.38 |
| 5 | 2-thionaphthol | 1.26 |

Examples 6-9

Tetralin was used as a hydrogen donor with benzophenone as a model hydrogen acceptor in the manner described in Examples 2-5, using the same catalysts. The results are given in Table 2 below.

Table 2

Tetralin with Sulfur H-Transfer Catalysts

| Example | Catalyst | HDonEff |
|---|---|---|
| 6 | $n\text{-}C_{12}H_{25}SH$ | 1.41 |
| 7 | thiophenol | 2.84 |
| 8 | 1-thionaphthol | 1.23 |
| 9 | 2-thionaphthol | 2.46 |

Examples 10 and 11

The effectiveness of heterocyclic compounds as H-donors was shown by using indoline and piperidine in the manner described in Examples 2-5, using benzophenone as the H-acceptor and thiophenol as the catalyst. Results are given in Table 3 below.

Table 3

Heterocyclic H-Donors

| Example | H-Donor | HDonEff |
|---|---|---|
| 10 | Indoline | 5.33 |
| 11 | Piperidine | 4.94 |

4

**0 195 541**

**Claims**

1. A process for the conversion of an alicyclic compound into an aromatic compound which comprises dehydrogenating the alicyclic compound in the presence of a hydrogen acceptor and a catalyst, characterized in that the catalyst is an organic, active sulfur-containing compound.

2. A process according to claim 1, which is carried out at an elevated temperature.

3. A process according to claim 1 or claim 2, which is carried out in the liquid phase.

4. A process according to any one of claims 1 to 3, in which the alicyclic compound contains multiple carbocyclic rings.

5. A process according to claim 4, in which the alicyclic compound is decahydronaphthalene.

6. A process according to any one of claims 1 to 3, in which the alicyclic compound contains a heterocyclic ring.

7. A process according to any one of claims 1 to 6, in which the hydrogen acceptor comprises a bis-aromatic ketone.

8. A process according to any one of claims 1 to 7, in which the active sulfur-containing compound is a thiol or thiophenol.

9. A process according to any one of claims 1 to 8, in which the active sulfur compound or a precursor of it is contained in a petroleum refinery process stream.

**Patentansprüche**

1. Verfahren zur Umwandlung einer alizyklischen Verbindung in eine aromatische Verbindung, das die Dehydrierung dieser alizyklischen Verbindung in Gegenwart eines Wasserstoffakzeptors und eines Katalysators umfaßt, dadurch gekennzeichnet, daß der Katalysator eine organische, aktive schwefelhaltige Verbindung ist.

2. Verfahren nach Anspruch 1, das bei erhöhter Temperatur durchgeführt wird.

3. Verfahren nach Anspruch 1 oder 2, das in der flüssigen Phase durchgeführt wird.

4. Verfahren nach einem der Ansprüche 1 bis 3, worin die alizyklische Verbindung mehrfach carbozyklische Ringe enthält.

5. Verfahren nach Anspruch 4, worin die alizyklische Verbindung Dekahydronaphthalin ist.

6. Verfahren nach einem der Ansprüche 1 bis 3, worin die alizyklische Verbindung einen heterozyklischen Ring enthält.

7. Verfahren nach einem der Ansprüche 1 bis 6, worin der Wasserstoffakzeptor ein bis-aromatisches Keton umfaßt.

8. Verfahren nach einem der Ansprüche 1 bis 7, worin die aktive schwefelhaltige Verbindung ein Thiol oder Thiophenol ist.

9. Verfahren nach einem der Ansprüche 1 bis 8, worin die aktive Schwefelverbindung oder eine Vorstufe davon in einem Erdölraffinerie-Verfahrensstrom enthalten ist.

**Revendications**

1. Un procédé de conversion d'un dérivé alicyclique en un dérivé aromatique qui consiste à déshydrogéner le dérivé alicyclique en présence d'un accepteur d'hydrogène et d'un catalyseur, caractérisé en ce que le catalyseur est un dérivé organique contenant du soufre actif.

2. Un procédé selon la revendication 1, qui est mis en œuvre à température élevée.

3. Un procédé selon la revendication 1 ou 2, qui est mis en œuvre en phase liquide.

4. Un procédé selon l'une quelconque des revendications 1 à 3, dans lequel le dérivé alicyclique contient des cycles carbocycliques multiples.

5. Un procédé selon la revendication 4, dans lequel le dérivé alicyclique est le décahydronaphtalène.

6. Un procédé selon l'une quelconque des revendications 1 à 3, dans lequel le dérivé alicyclique contient un cycle hétérocyclique.

7. Un procédé selon l'une quelconque des revendications 1 à 6, dans lequel l'accepteur d'hydrogène comprend une cétone bis-aromatique.

8. Un procédé selon l'une quelconque des revendications 1 à 7, dans lequel le dérivé contenant du soufre actif est un thiol ou un thiophénol.

9. Un procédé selon l'une quelconque des revendications 1 à 8, dans lequel le dérivé de soufre actif ou un de ses précurseurs est contenu dans un courant provenant d'un procédé de raffinage de pétrole.